# EUROPEAN PATENT APPLICATION

(11) **EP 4 318 483 A1**
(43) Date of publication of application: **07.02.2024**
(21) Application number: 22775847.1
(22) Date of filing: 25.03.2022
(51) Int. Cl.: G16H 10/60, G16H 15/00, G16H 20/10

(54) **DRUG-DISPENSATION INFORMATION MANAGING DEVICE, MANAGING METHOD, AND PROGRAM RECORDING MEDIUM**

(30) Priority: 26.03.2021 JP 2021054090
(71) Applicant: NTT Communications Corporation, Tokyo 100-8019 (JP)
(72) Inventor: SAKURAI, Yoichi, Tokyo 100-8019 (JP); UMEDA, Takeru, Tokyo 100-8019 (JP); KOKUBO, Ryuta, Tokyo 100-8019 (JP); OTA, Mitsunori, Tokyo 100-8019 (JP); IZUMI, Hiroaki, Tokyo 100-8019 (JP)
(74) Representative: Gramm, Lins & Partner Patent- und Rechtsanwälte PartGmbB
(86) International application number: PCT/JP2022/014624
(87) International publication number: WO 2022/203066

(57) **Abstract**

Verification of not only medication completion information but also information indicating post-medication conditions can be realized. The medication completion information indicating the fact of the medication having been administered to a target recipient is obtained, and the obtained medication completion information is stored in association with the unique identification information of the target recipient. The post-medication information indicating the condition of the target recipient after the medication is obtained from the user terminal, and the obtained post-medication information is stored in association with the medication completion information. Medication certification information is created based on the stored medication completion information and post-medication information, and the process for submitting the created medication certification information from the target recipient to an organization is controlled.

## Description

### FIELD

One aspect of the present invention relates to a medication information management apparatus, a management method, and a program storage medium for managing information that relates to medications such as vaccines.

### BACKGROUND

Vaccination is particularly effective as a preventive measure against epidemic diseases. When vaccination is conducted, however, the record of the vaccination is only entered into a chart of a medical institution or the like, and a certification relating to a vaccination status is not issued.

On the other hand, for domesticated animals or pet animals, a system has been proposed in which a certification of mandatory vaccination is registered in a server together with individual identification information so that the certification of vaccination can be obtained as needed from the server (see Patent Literature 1, for example).

### CITATION LIST

### PATENT LITERATURE

PATENT LITERATURE 1: Jpn. PCT National Publication NO. 2012-506595

### SUMMARY

### TECHNICAL PROBLEM

With the technique described in Patent Literature 1, a veterinarian or the owner of a domesticated animal simply inputs the record of the vaccination in a predetermined format and stores the record in the server so that this vaccination record can be used as-is as certification. From the vaccination record, only the completion of vaccination can be grasped, and a change or the like in the condition of an individual after vaccination cannot be ascertained. Furthermore, there is a possibility that an unauthorized change, such as tampering, may be added to an issued vaccination certification, but countermeasures against such a possibility is not fully considered. For this reason, a reliability issue remains in connection with vaccination certifications.

The present invention has been made in view of the above circumstances. An object of the present invention is to provide a technique that realizes issuance of a medication certification that contains not only information indicating the completion of medication but also information indicating a post-medication condition.

### SOLUTION TO PROBLEM

In order to solve the above issue, the first aspect of the present invention relates to a medication information management apparatus or a medication information management method capable of transmitting information data via a network between a user terminal that is used by a target recipient of the medication and an organization terminal that is used by an organization to which the medication certification information relating to the medication is to be submitted.

A management apparatus or a management method according to the first aspect of the present invention obtains medication completion information that indicates the medication administered to the target recipient, stores the obtained medication completion information in association with unique identification information of the target recipient, obtains post-medication information indicating the condition of the target recipient after the medication from the user terminal, and stores the obtained post-medication information in association with the medication completion information. A submission process for creating the medication certification information on the basis of the medication completion information and the post-medication information and submitting the created medication certification information from the target recipient to the organization is controlled.

According to the first aspect of the present invention, for example, with respect to the target recipient who has received the medication, the post-medication information indicating the condition of the target recipient after the medication is obtained in addition to the medication completion information indicating the fact of the medication having been administered, and the medication certification information in which both the medication completion information and the post-medication information are reflected, is created. Then, the submission process is controlled at the time of the target recipient submitting the created medication certification information to a submission target organization. As a result, it is possible to issue, for a target recipient who has received the medication, a certification that includes not only the medication completion information indicating the fact of the medication having been administered but also the post-medication information indicating a change in the condition such as adverse reactions of the target recipient after the medication.

According to the second aspect of the present invention, in the control of the submission process of the medication certification information, a verification procedure of the submitted medication certification information is additionally executed. As a result, it is possible to verify whether or not an unauthorized change such as tampering has been made to the medication certification information, that is, whether or not the medication certification information is authentic, so that a high reliability of the medication certification information can be maintained.

### ADVANTAGEOUS EFFECTS OF INVENTION

According to one aspect of the present invention, it is possible to provide a technique for issuing a medication certification that includes not only information indicating the completion of the medication but also information indicating a post-medication condition.

### BRIEF DESCRIPTION OF THE DRAWINGS

FIG. 1 is a diagram showing an exemplary configuration of the entire system that includes a medication information management apparatus according to a first embodiment of the present invention.
FIG. 2 is a block diagram showing an exemplary hardware configuration of a vaccine recipient terminal used in the system shown in FIG. 1.
FIG. 3 is a block diagram showing an exemplary software configuration of the vaccine recipient terminal shown in FIG. 2.
FIG. 4 is a block diagram showing an exemplary hardware configuration of a vaccination information management server included in the medication information management apparatus shown in FIG. 1.
FIG. 5 is a block diagram showing an exemplary software configuration of the vaccination information management server shown in FIG. 4.
FIG. 6 is a block diagram showing an exemplary software configuration of a vaccination certification management server included in the medication information management apparatus shown in FIG. 1.
FIG. 7 is a flowchart showing an exemplary procedure and description of the process conducted by the control unit of the vaccine recipient terminal shown in FIG. 3.
FIG. 8 is a flowchart showing an exemplary procedure and description of the process conducted by the control unit of the vaccination information management server shown in FIG. 5.
FIG. 9 is a flowchart showing an exemplary procedure and description of the process conducted by the control unit of the vaccination certification management server shown in FIG. 6.
FIG. 10 is a sequence diagram illustrating an exemplary flow of the overall process in the system illustrated in FIG. 1.
FIG. 11 is a sequence diagram showing a flow of a vaccination certification confirmation process in the process of FIG. 10.
FIG. 12 is a sequence diagram showing a flow of a vaccination certification confirmation process according to a second embodiment of the present invention.

### DETAILED DESCRIPTION

Embodiments of the present invention will be described below with reference to the drawings.

### <First Embodiment>

### (Exemplary Configuration)

### (1) System

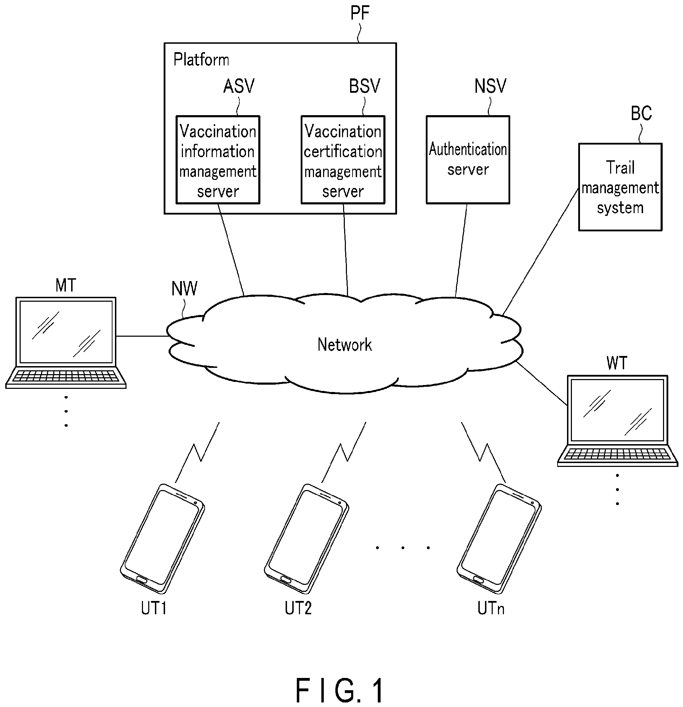

FIG. 1 is a diagram showing an exemplary configuration of the entire system including a medication information management apparatus PF according to the first embodiment of the present invention. Hereinafter, the medication information management apparatus PF may also be referred to as a platform.

The platform PF may be provided on a cloud, and it includes, in this example, a vaccination information management server ASV and a vaccination certification management server BSV. An authentication server NSV and a trail management system BC can be connected to the platform PF via a network NW.

The platform PF may be configured to further include at least one of the authentication server NSV and the trail management system BC in addition to the vaccination information management server ASV and the vaccination certification management server BSV. The platform PF may be configured as a group of servers, or may be configured by a single server.

The platform PF realizes information data transmission via a network NW between a plurality of vaccine recipient terminals UT1 to UTn (hereinafter collectively referred to as UT) used by vaccine recipients, a medical service terminal MT used by medical workers such as doctors, and an organization terminal WT used by an organization to which the vaccine recipient's vaccination certification data will be submitted. As a medical service terminal MT, a personal computer may be used, and as an organization terminal WT, a personal computer or a Point of Sale (POS) terminal may be used.

The authentication server NSV executes an authentication process on a vaccine recipient with the vaccine recipient terminal UT. For example, a vaccine recipient is identified using Japanese Public Key Infrastructure (JPKI), and then authentication information that includes an authentication ID and a password may be set up. In addition, unique identification information of the vaccine recipient is issued and set in the vaccination information management server ASV and the trail management system BC. Further, an address is set in the trail management system BC to allow the vaccine recipient to use this trail management system BC, and a secret key associated with this address is issued and provided to the vaccine recipient terminal UT.

The trail management system BC is configured, for example, by a blockchain platform, in which a plurality of distributed ledgers are connected via a peer-to-peer(P2P) network. Every time the vaccination certification data of a vaccine recipient is issued at the vaccination certification management server BSV, which will be described later, the trail management system BC generates a token having management information of each piece of the individual vaccination certification data as an attribute value by a contract in response to an instruction from the vaccination certification management server BSV, and stores the generated token as a transaction. Furthermore, every time the issued vaccination certification data is used, the trail management system BC updates the attribute value of the corresponding token by the contract and stores the information indicating the history of changes as a transaction.

Upon receipt of a verification request of a token for a piece of vaccination certification data from the organization terminal WT, the trail management system BC also conducts verification regarding whether or not an unauthorized change such as tampering has been made to this vaccination certification data, based on the hash value included in the stored token.

The network NW includes, for example, a wide area network designed around the Internet and an access network for accessing this wide area network. Examples of the access network include a public communication network using wired or wireless communications, a local area network (LAN) using wired or wireless communications, and a cable television (CATV) network.

### (2) Devices

### (2-1) Vaccine recipient terminal UT

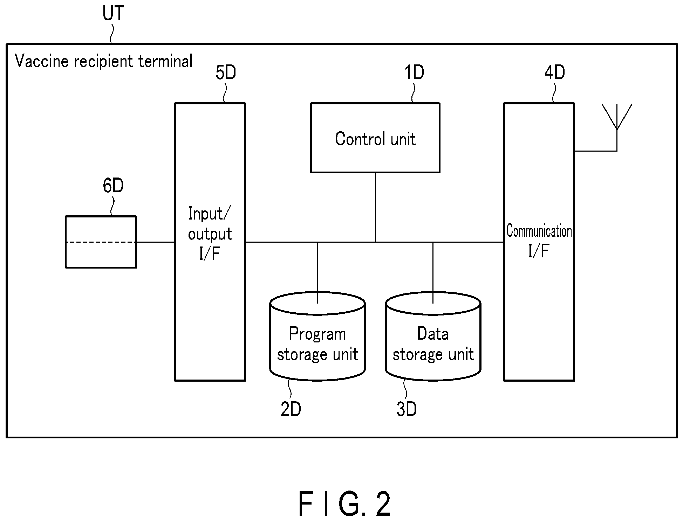

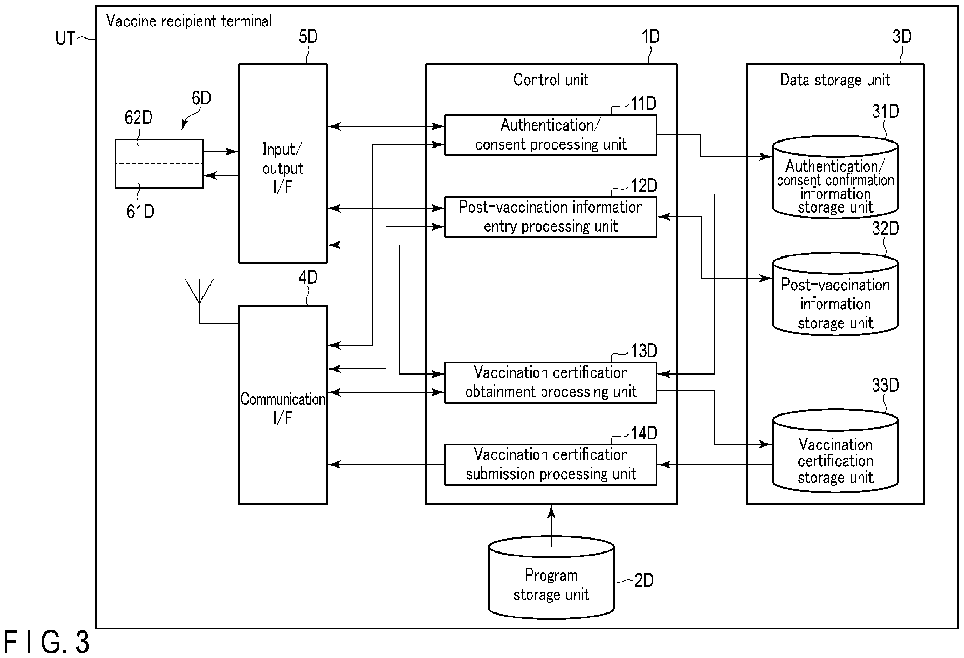

FIGS. 2 and 3 are block diagrams showing an exemplary hardware configuration and software configuration, respectively, of a vaccine recipient terminal UT.

The vaccine recipient terminal UT is a general-purpose smartphone having, for example, a browser and a function of transferring information data, such as electronic mails, social network system (SNS), and short message service (SMS). Alternatively, a tablet terminal, a notebook personal computer, or the like that has a similar function may be used as a vaccine recipient terminal UT

A vaccine recipient terminal UT includes a control unit 1D, which adopts a hardware processor such as a central processing unit (CPU). The control unit 1D is connected to a storage unit including a program storage unit 2D and a data storage unit 3D, a communication interface (hereinafter "interface" will be referred to as "I/F") 4D, and an input/output I/F 5D.

The communication I/F 4D transmits information data under the control of the control unit 1D between the vaccination information management server ASV and the vaccination certification management server BSV in the platform PF and also between the authentication server NSV and the trail management system BC, using a communication protocol defined by the network NW, for example, Transmission Control Protocol/Internet Protocol (TCP/IP). The communication I/F 4D may include an interface corresponding to a low-power wireless data communication standard such as Bluetooth (trademark) in order to perform data transfer, for example, to and from an organization terminal WT.

The input/output I/F 5D is connected to an input/output device 6D. The input/output device 6D may be configured by arranging an input unit 62D, which adopts a touch input sheet of a pressure-sensitive type or a capacitance type, upon the display screen of a display unit 61D, which adopts a liquid crystal type or an organic EL type. The input unit 62D is used by a vaccine recipient to input information necessary for his/her own authentication, post-vaccination information relating to adverse reactions and the like, and commands and information data necessary for obtainment and submission of the vaccination certification data. The display unit 61D is configured to display the above-mentioned commands, various kinds of information data, the obtained vaccination certification data, and the like.

In addition to the above, a camera, a global positioning system (GPS) sensor for finding the position of the vaccine recipient, and a vital sensor for measuring biological information such as the temperature, blood pressure, and heart rate of the vaccine recipient may be connected to the input/output I/F 5D.

The program storage unit 2D is configured by combining a non-volatile memory upon which writing and reading can be performed at any time, such as a Solid State Drive (SSD), and a non-volatile memory such as a Read Only Memory (ROM) as storage media so as to store multiple application programs necessary for executing various control processes according to the first embodiment, in addition to middleware such as an operating system (OS). Hereinafter, the middleware such as the OS and the application programs will be collectively referred to as programs.

The data storage unit 3D is configured by combining a non-volatile memory, such as an SSD, upon which writing and reading can be performed at any time, and a volatile memory, such as a Random Access Memory (RAM). The data storage unit 3D includes an authentication/consent confirmation information storage unit 31D, a post-vaccination information storage unit 32D, and a vaccination certification storage unit 33D as main storage units necessary for performing the process according to the first embodiment.

The authentication/consent confirmation information storage unit 31D is configured to store a secret key associated with the vaccine recipient address for the trail management system BS, which is provided by the authentication server NSV at the time of initial setup of the authentication information of the vaccine recipient with the authentication server NSV.

The post-vaccination information storage unit 32D is configured to temporarily store the post-vaccination information that has been input from the input unit 62D in relation to adverse reactions and the like of the vaccine recipient.

The vaccination certification storage unit 33D is configured to store the vaccination certification data obtained from the vaccination certification management server BSV. The vaccination certification data is represented by code data such as a bar code or a QR code (trademark), or may be represented by text data or binary data.

As processing functions necessary to implement the first embodiment, the control unit 1D includes an authentication/consent processing unit 11D, a post-vaccination information entry processing unit 12D, a vaccination certification obtainment processing unit 13D, and a vaccination certification submission processing unit 14D. Each of these processing units 11D to 14D is realized by causing the hardware processor of the control unit 1D to execute a program stored in the program storage unit 2D.

The authentication/consent processing unit 11D executes an authentication procedure or a consent confirmation procedure for a vaccine recipient with the authentication server NSV at the time of initial setup of a vaccine recipient terminal UT, at the time of entering the post-vaccination information, or at the time of obtaining a vaccination certification. At the initial setup, a process is implemented such that a secret key associated with the vaccine recipient address that has been set up in the trail management system BS by the authentication server NSV will be received from the authentication server NSV and stored in the authentication/consent confirmation information storage unit 31D.

Upon receipt of a post-vaccination information obtainment request from the vaccination information management server ASV, the post-vaccination information entry processing unit 12D temporarily stores in the post-vaccination information storage unit 32D the post-vaccination information relating to the adverse reactions and the like that has been input by the vaccine recipient on the authentication input unit 62D. Thereafter, a process for transmitting the stored post-vaccination information from the communication I/F 4D to the vaccination information management server ASV is executed.

Upon manipulation of the input unit 62D by the vaccine recipient to request the obtainment of vaccination certification, the vaccination certification obtainment processing unit 13D executes a process for obtaining the vaccination certification data with the vaccination certification management server BSV via the communication I/F 4D. Thereafter, the obtained vaccination certification data is stored in the vaccination certification storage unit 33D.

If the vaccine recipient manipulates the input unit 62D to perform an operation for submitting the vaccination certification data to an organization, the vaccination certification submission processing unit 14D executes a process for reading the vaccination certification data from the vaccination certification storage unit 33D and outputting the data. For this output means, two schemes are possible. One scheme is to display, if the vaccination certification data is represented as code data such as a bar code or a QR code (trademark), this code data on the display unit 61D. The other scheme is to transmit, if the vaccination certification data is represented by text data or binary data, this text data or binary data wirelessly from the communication I/F 4D to the organization terminal WT, to which the vaccination certification data will be submitted.

### (2-2) Vaccination information management server ASV

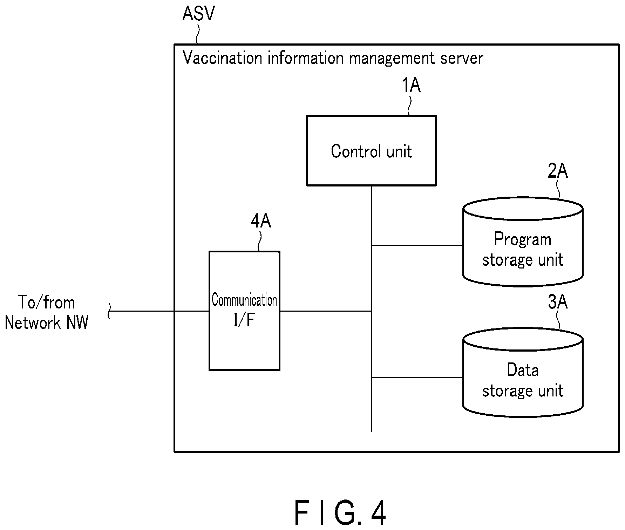

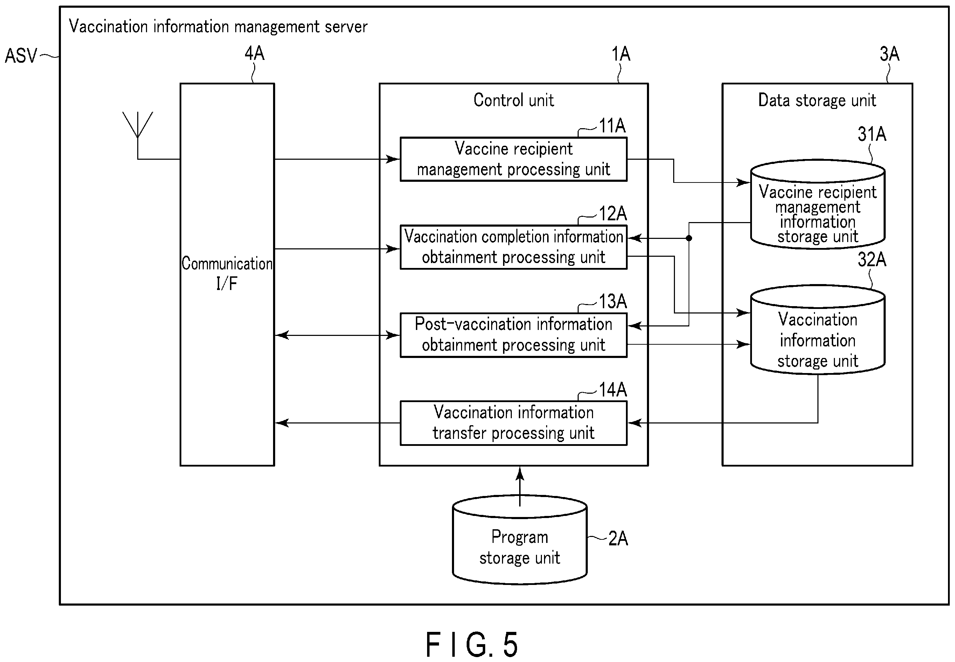

FIGS. 4 and 5 are block diagrams showing exemplary hardware configuration and software configuration, respectively, of the vaccination information management server ASV.

The vaccination information management server ASV may be a server computer, and includes a control unit 1A, which adopts a hardware processor such as a CPU. This control unit 1A is connected to a storage unit having a program storage unit 2A and a data storage unit 3A and to a communication I/F 4A via a bus.

Under the control of the control unit 1A, the communication I/F 4A transmits and receives information data to and from the vaccination certification management server BSV in the platform PF, and also to and from the vaccine recipient terminals UT and the medical service terminals MT, using a communication protocol defined by the network NW. The communication I/F 4A can also transmit and receive information data to and from the authentication server NSV and the trail management system BC.

The program storage unit 2A is configured by combining, as a storage medium, a non-volatile memory such as a hard disk drive (HDD) or an SSD, upon which writing and reading can be performed at any time, and a non-volatile memory such as a ROM, so as to store programs necessary to execute various control processes according to the first embodiment of the present invention, in addition to middleware such as an OS.

The data storage unit 3A is configured by combining, as a storage medium, a non-volatile memory such as an HDD or an SSD, upon which writing and reading can be performed at any time, and a volatile memory such as a RAM. As main storage units necessary to implement the first embodiment of the present invention, the data storage unit 3A includes a vaccine recipient management information storage unit 31A and a vaccination information storage unit 32A.

The vaccine recipient management information storage unit 31A is configured to store basic information and medical inquiry information of each vaccine recipient. The vaccination information storage unit 32A is configured to store the vaccination completion information and the post-vaccination information of the vaccine recipients obtained from the medical service terminal MT and the vaccine recipient terminals UT.

The control unit 1A includes, as processing functions according to the first embodiment of the present invention, a vaccine recipient management processing unit 11A, a vaccination completion information obtainment processing unit 12A, a post-vaccination information obtainment processing unit 13A, and a vaccination information transfer processing unit 14A. Each of these processing units 11A to 14A is realized by causing the hardware processor of the control unit 1A to execute a program stored in the program storage unit 2A.

Prior to the vaccination, the vaccine recipient management processing unit 11A receives the basic information of a vaccine recipient sent from the vaccine recipient terminal UT via the communication I/F 4A and stores the received basic information in the vaccine recipient management information storage unit 31A in association with the unique identification information of the vaccine recipient.

The vaccination completion information obtainment processing unit 12A receives the vaccination completion information, which is sent from the medical service terminal MT at the time of vaccination of the vaccine recipient, together with the medical inquiry information via the communication I/F 4A, and stores the received vaccination completion information together with the medical inquiry information in the vaccination information storage unit 32A in association with the unique identification information of the vaccine recipient.

The vaccination completion information may include the type of vaccine administered, the identification information of the pharmaceutical company (manufacturer ID), and the lot number, and may additionally include a vaccine recipient identification number, a vaccination date and time, a vaccination site, and the like.

The medical inquiry information includes, but is not limited to, information indicating the temperature of the vaccine recipient immediately before the vaccination, pre-existing conditions, current medication types, drug allergy status, pregnancy status, changes in physical condition in a recent predetermined period, and the like.

If the vaccine recipient terminal UT obtains and manages the personal health record (PHR) information of the vaccine recipient and prescription/medication information described in an electronic medication record or the like, such information may be obtained together with the medical inquiry information and stored in the vaccine recipient management information storage unit 31A. The PHR information may include vital data such as the current body temperature, blood pressure, and heart rate measured by a biosensor, which is built in or attached to the vaccine recipient terminal UT.

The post-vaccination information obtainment processing unit 13A transmits a post-vaccination information report request to the vaccine recipient terminals UT of the vaccine recipients for whom vaccination completion information has been entered, at a post-vaccination information obtainment timing after a predetermined period has elapsed since the vaccination. The post-vaccination information obtainment processing unit 13A receives the post-vaccination information returned from the vaccine recipient terminals UT via the communication I/F 4A, and stores the received post-vaccination information in the vaccination information storage unit 32A in association with the unique identification information (vaccine recipient ID) of the vaccine recipients.

Upon receipt of a vaccination information obtainment request from the vaccination certification management server BSV, the vaccination information transfer processing unit 14A reads from the vaccination information storage unit 32A the vaccination information, i.e., the vaccination completion information and the post-vaccination information, of the corresponding vaccine recipient, and transfers the read-out information from the communication I/F 4A to the vaccination certification management server BSV of the request source. Here, the vaccination information transfer processing unit 14A may transfer the basic information of the corresponding vaccine recipient stored in the vaccine recipient management information storage unit 31A, together with the vaccination completion information and the post-vaccination information, to the vaccination certification management server BSV of the request source.

### (2-3) Vaccination certification management server BSV

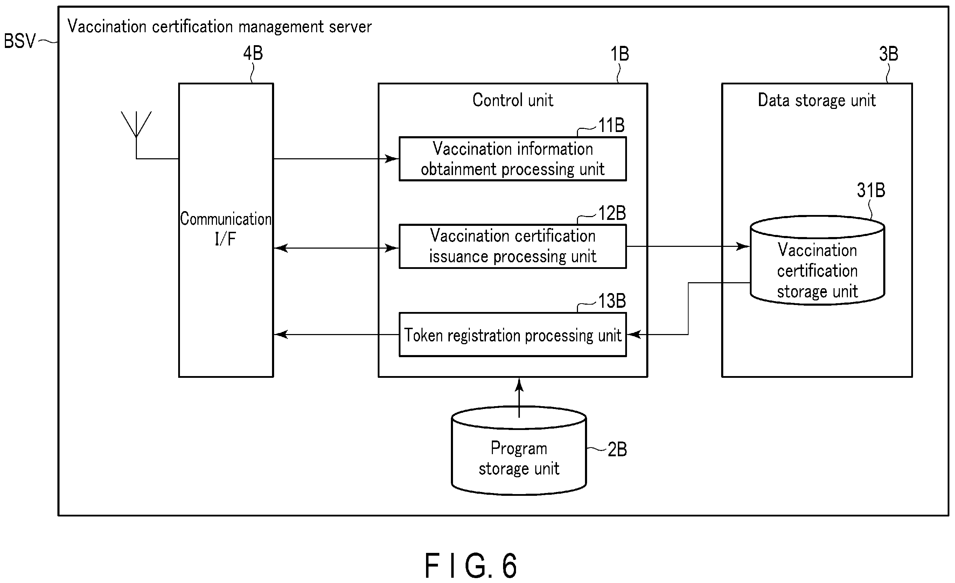

FIG. 6 is a block diagram showing a software configuration of the vaccination certification management server BSV. Since the hardware configuration thereof is the same as the configuration of the vaccination information management server ASV (FIG. 4), the description will be omitted here.

The vaccination certification management server BSV includes a control unit 1B, a program storage unit 2B, a data storage unit 3B, and a communication I/F 4B. The communication I/F 4B transmits and receives information data to and from the vaccination information management server ASV in the platform PF, and also to and from the trail management system BC, the vaccine recipient terminals UT, and the organization terminal WT via the network NW.

A vaccination certification storage unit 31B is provided in the data storage unit 3B. The vaccination certification storage unit 31B is configured to store the vaccination certification data generated by the control unit 1B in association with the unique identification information of the vaccine recipient (vaccine recipient ID) .

The control unit 1B includes a vaccination information obtainment processing unit 11B, a vaccination certification issuance processing unit 12B, and a token registration processing unit 13B as processing functions according to the first embodiment of the present invention. These processing units 11B to 13B are realized by causing the hardware processor of the control unit 1B to execute a program stored in the program storage unit 2B.

Upon receipt of a vaccination certification obtainment request from a vaccine recipient terminal UT or an organization terminal WT, the vaccination information obtainment processing unit 11B obtains the vaccination completion information and the post-vaccination information of the corresponding vaccine recipient from the vaccination information management server ASV and transfers the obtained information to the vaccination certification issuance processing unit 12B.

The vaccination certification issuance processing unit 12B creates vaccination certification data based on the vaccination completion information and the post-vaccination information received from the vaccination information obtainment processing unit 11B, and temporarily stores the created vaccination certification data in the vaccination certification storage unit 31B in association with the unique identification information of the vaccine recipient. The vaccination certification issuance processing unit 12B determines whether or not the vaccine recipient agrees to a third party's use of the vaccination certification data on the basis of the information of a consent form managed by the authentication server NSV. Upon confirmation of the consent, the vaccination certification issuance processing unit 12B transmits the vaccination certification data to the vaccine recipient terminal UT or organization terminal WT of the request source.

The token registration processing unit 13B instructs the contract of the trail management system BC to generate or update a token having the management information of the vaccination certification data as an attribute value at the time of issuance or use (e.g., transfer) of the vaccination certification data and change of the attribute value.

### (Exemplary Operation)

Next, an exemplary operation of the above configured system will be described.

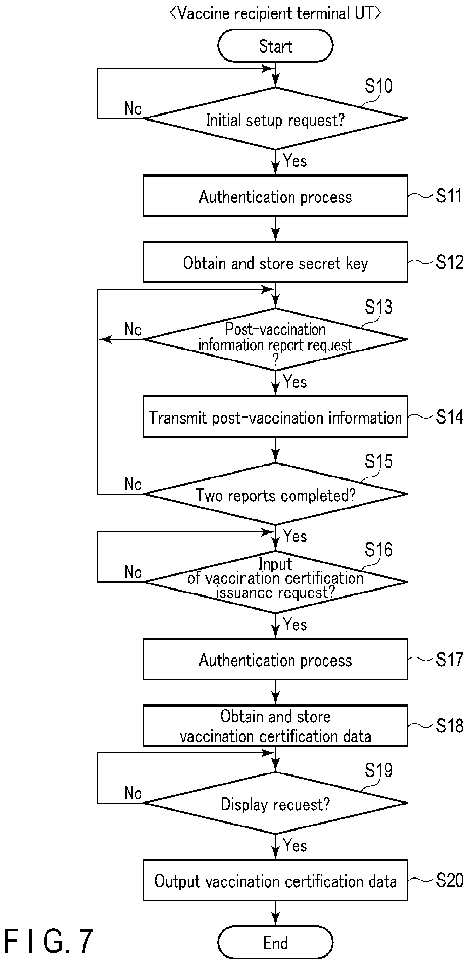

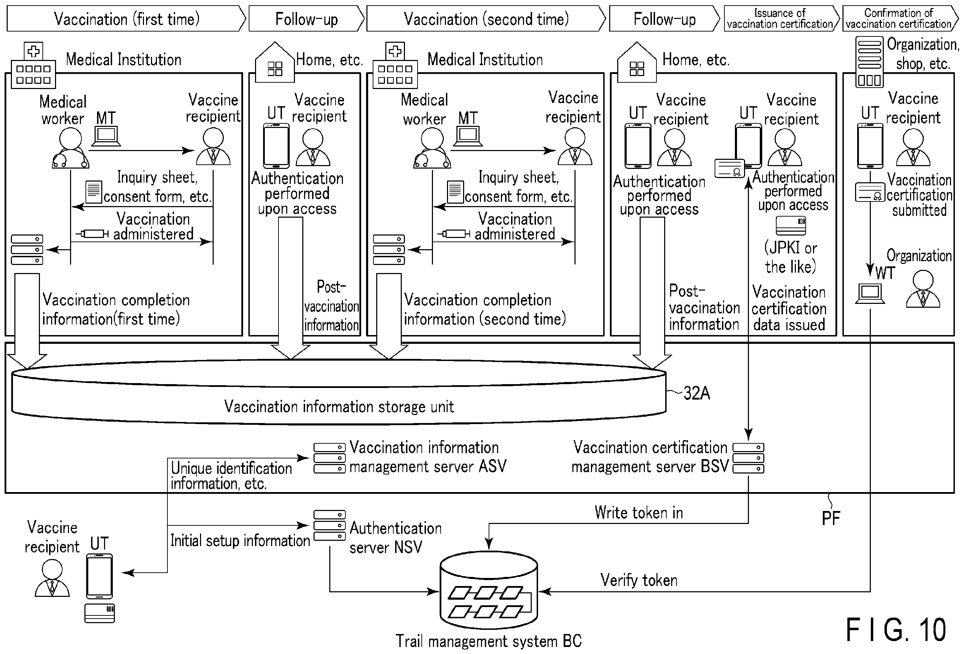

FIGS. 7 to 9 are flowcharts showing the procedures and description of the process conducted by the vaccine recipient terminal UT, the vaccination information management server ASV, and the vaccination certification management server BSV, respectively. FIG. 10 is a sequence diagram showing the processing flow of the entire system.

### (1) Initial setup of vaccine recipient terminal UT

Prior to the vaccination, if a person who is going to receive vaccination accesses the authentication server NSV from his/her vaccine recipient terminal UT, authentication information is set up between the vaccine recipient terminal UT and the authentication server NSV, as described below.

As shown in FIG. 7, at step S10, upon detection of an input of a request for setup of the processing registration by the vaccine recipient, the control unit 1D of the vaccine recipient terminal UT performs an initial setup process of the authentication information with the authentication server NSV under the control of the authentication/consent processing unit 11D at step 511. In this process, the authentication/consent processing unit 11D identifies the vaccine recipient by using, for example, Japanese Public Key Infrastructure (JPKI), and then receives an input of an authentication ID and a password from the vaccine recipient.

In response to the above, the authentication server NSV issues the unique identification information of the vaccine recipient (vaccine recipient ID) and reports it to the vaccination information management server ASV and the trail management system BC. Further, the authentication server NSV sets up an address for the vaccine recipient to use the trail management system BC and a secret key associated with this address, and informs the vaccine recipient terminal UT of the secret key. The control unit 1D of the vaccine recipient terminal UT stores the received secret key in the authentication/consent confirmation information storage unit 31D at step S12.

After the initial setup of the vaccine recipient in the authentication server NSV, the control unit 1A of the vaccination information management server ASV proceeds from step S30 to step S31, where it obtains the unique identification information of the vaccine recipient from the authentication server NSV and stores it in the vaccine recipient management information storage unit 31A. The trail management system BC receives the address of the target vaccine recipient initially set up by the authentication server ASV and sets the address in the distributed ledger.

### (2) Registration of basic information of vaccine recipient

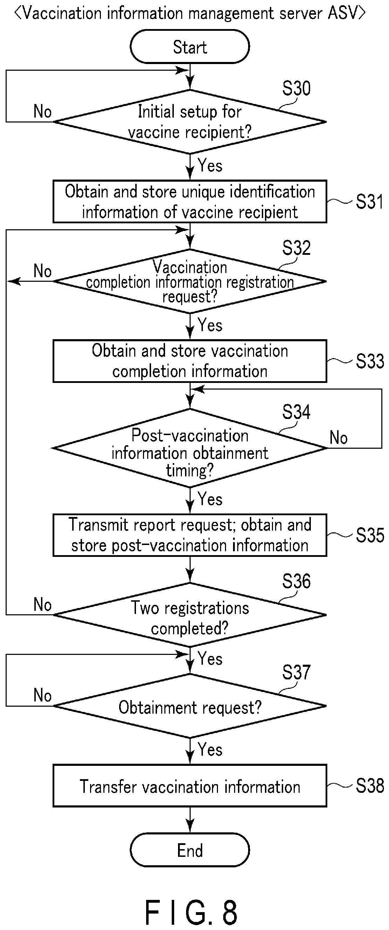

After completion of the initial setup, the vaccine recipient registers his/her own basic information from the vaccine recipient terminal UT upon the platform PF prior to the vaccination. In this case, for example, as shown in FIG. 8, at step S30, upon receipt of a registration request of the basic information from the vaccine recipient terminal UT, the vaccination information management server ASV of the platform PF receives the basic information transmitted from the vaccine recipient terminal UT via the communication I/F 4A under the control of the vaccine recipient management processing unit 11A at step S31. The received basic information is stored in the vaccine recipient management information storage unit 31A in association with the unique identification information of the vaccine recipient.

The basic information includes, for example, information carried on a vaccination card sent in advance from a local government or the like, such as the identification information of the target vaccine recipient managed by an administrative organization of a local government or the like, a designated vaccination date and time or period, a vaccination site, a type of vaccine to be administered, and the like. The information on this vaccination card may be presented by a QR code, and is read by a camera and a code recognition application included in the vaccine recipient terminal UT. As the basic information, attribute information of the vaccine recipient, such as contact information including his/her name, age, address, telephone number or electronic mail address, may also be registered. Such attribute information may be registered by the vaccine recipient inputting it on the vaccine recipient terminal UT.

### (3) Registration of vaccination completion information associated with first vaccination

To receive a vaccination, the target vaccine recipient first fills out an inquiry sheet and a consent form, and submits them to the medical worker. If the medical worker examines the content of the submitted inquiry sheet and determines that vaccination can be conducted, the medical worker administers vaccination to the target vaccine recipient. After the vaccination, the medical worker inputs the vaccination completion information on the medical service terminal MT. The vaccination completion information includes, for example, the type of vaccine administered, the identification information of the pharmaceutical company (manufacturer ID), and the lot number, and it may further include a vaccine recipient identification number, a vaccination date and time, and a vaccination site.

The medical worker further inputs to the medical service terminal MT the inquiry sheet information and the consent form submitted by the vaccine recipient. The input of the information on this inquiry sheet and consent form may be performed, for example, by reading with an optical character reader (OCR).

As mentioned earlier, the inquiry sheet information includes, but is not limited to, information indicating the body temperature of the target vaccine recipient immediately before the vaccination, pre-existing conditions, current medication types, drug allergy status, pregnancy status, changes in physical condition in a recent predetermined period, and the like.

The consent form information includes information indicating whether or not to agree to a third party's use of the vaccination certification data of the vaccine recipient, but it is not limited thereto. The consent form information may include, for example, information indicating whether or not to agree to the use of the post-vaccination information by a pharmaceutical company, medical institution, local government, or the like.

As means for inputting the inquiry sheet information and the consent form information, the vaccine recipient terminal UT or an information input device such as a tablet terminal provided in a vaccination site may be employed.

A medical worker may input from the medical service terminal MT the information of the vaccination card submitted by the target vaccine recipient together with the inquiry sheet information and the consent form information. In this manner, even if the vaccine recipient does not have a vaccine recipient terminal UT, the information of the vaccination card can be input.

From among the entered vaccination completion information, inquiry sheet information, and consent form information, the medical service terminal MT transmits the vaccination completion information and the inquiry sheet information to the vaccination information management server ASV of the platform PF. If the information of the vaccination card is entered along with these, this information is also transmitted to the vaccination information management server ASV.

Upon receipt of a registration request of the vaccination completion information and the like at step S32, the control unit 1A of the vaccination information management server ASV receives the vaccination completion information and the inquiry sheet information, as well as the information of the vaccination card transmitted from the medical service terminals MT, via the communication I/F 4A under the control of the vaccination completion information obtainment processing unit 12A at step S33. The received information is stored in the vaccination information storage unit 32A in association with the unique identification information of the vaccine recipient.

In this manner, the vaccination completion information for the first vaccination of the vaccine recipient is registered together with the inquiry sheet information and the information of the vaccination card in the vaccination information management server ASV of the platform PF.

On the other hand, among the information of the vaccination completion, the inquiry sheet, and the consent form, the consent form information is transmitted from the medical service terminal MT to the authentication server NSV. The authentication server NSV stores and manages the consent form information together with the authentication information in association with the unique identification information of the vaccine recipient.

### (4) Registration of post-vaccination information after first vaccination

At the timing of obtaining the post-vaccination information after a predetermined period of time has elapsed since the first vaccination, the control unit 1A of the vaccination information management server ASV proceeds from step S34 to step S35 under the control of the post-vaccination information obtainment processing unit 13A, and transmits a post-vaccination information report request message from the communication I/F 4A to the target vaccine recipient terminal UT. This report request message may be transmitted, for example, by electronic mail, SNS, or SMS.

Upon receipt of this report request message at step S13, the control unit 1D of the vaccine recipient terminal UT displays the received report request message on the display unit 61D via the input/output I/F 5D under the control of the post-vaccination information entry processing unit 12D. In this situation, if the vaccine recipient accesses the uniform resource locator (URL) included in the displayed report request message, the authentication of the vaccine recipient is conducted based on the authentication information managed by the authentication server NSV, and then questionnaire data is downloaded from the vaccination information management server ASV and displayed on the display unit 61D. This questionnaire data includes multiple questions in order to ascertain the status of adverse reactions and the like after the vaccination.

In this situation, the vaccine recipient inputs answer data to each of the questions in the questionnaire data on the input unit 62D and presses the enter button. Then, the post-vaccination information entry processing unit 12D sends the answer data from the communication I/F 4D back to the vaccination information management server ASV. As means for obtaining this post-vaccination information, the electronic Patient Reported Outcomes (ePRO) technique may be adopted.

When the vaccine recipient terminal UT returns the answer data, the post-vaccination information obtainment processing unit 13A of the vaccination information management server ASV receives this answer data via the communication I/F 4A. At step S35, the answer data is stored in the vaccination information storage unit 32A as the post-vaccination information of this vaccine recipient in association with the unique identification information of the vaccine recipient.

In this manner, the post-vaccination information after the first vaccination is registered.

In the above exemplary operation, a report request message is transmitted from the vaccination information management server ASV to a vaccine recipient terminal UT, and in response, the vaccine recipient returns answer data. The configuration may be such that the reporting timing of the post-vaccination information is managed by the vaccine recipient on the vaccine recipient terminal UT and such that when the reporting timing arrives, the vaccine recipient terminal UT displays a message regarding this arrival on the display unit 61D so that the post-vaccination information input by the vaccine recipient will be transmitted from the vaccine recipient terminal UT to the vaccination information management server ASV. This will make it possible for the vaccine recipient to voluntarily report the post-vaccination information to the vaccination information management server ASV before a report request message is transmitted from the vaccination information management server ASV if there is a change in the condition of the vaccine recipient after the vaccination.

### (5) Registration of vaccination completion information associated with second vaccination

The registration process of the vaccination completion information associated with the second vaccination is performed in the same manner as the first vaccination. That is, the vaccine recipient visits the vaccination site on the vaccination date and time designated on the vaccination card, fills out an inquiry sheet and a consent form, and submits them to the medical worker. After the vaccine recipient receives the second vaccination, in a manner similar to the first vaccination, the vaccination completion information and the inquiry sheet information are transmitted from the medical service terminal MT to the platform PF, and registered in the vaccination information management server ASV of the platform PF. The consent form information is transmitted and registered in the authentication server NSV.

### (6) Registration of post-vaccination information after second vaccination

The registration process of the post-vaccination information after the second vaccination is performed in a manner similar to the first vaccination. That is, a post-vaccination report request message is transmitted from the vaccination information management server ASV to the vaccine recipient terminal UT after a predetermined period has elapsed since the vaccination. In this situation, if the vaccine recipient accesses the URL included in the report message, authentication of the vaccine recipient is conducted based on the authentication information managed by the authentication server NSV, and then questionnaire data is downloaded from the vaccination information management server ASV to the vaccine recipient terminal UT. When the vaccine recipient fills in the questionnaire data and enters the answer data, the answer data is sent back to the vaccination information management server ASV and is registered as post-vaccination information after the second vaccination in the vaccination information management server ASV.

### (7) Issuance of vaccination certification data

At step S15, the control unit 1D of the vaccine recipient terminal UT determines whether or not the process of entering the post-vaccination information after the second vaccination has been completed, and if it has, the control unit 1D monitors input of a vaccination certification obtainment request at step S16.

In this situation, if a vaccine recipient inputs a request for issuance of a vaccination certification on the vaccine recipient terminal UT in order to obtain his/her own vaccination certification data, the control unit 1D of the vaccine recipient terminal UT first executes, under the control of the authentication/consent processing unit 11D, a personal authentication process with the authentication server NSV at step S17. If the personal authentication is confirmed, a vaccination certification issuance request is transmitted to the vaccination certification management server BSV under the control of the vaccination certification obtainment processing unit 13D at step S18.

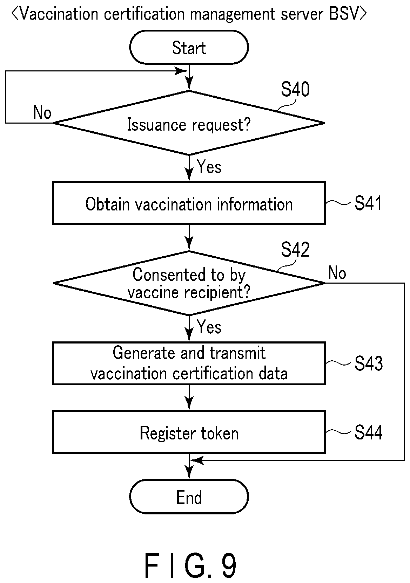

As shown in FIG. 9, the control unit 1B of the vaccination certification management server BSV monitors the reception of a vaccination certification issuance request at step S40. In this situation, when a vaccination certification issuance request is received, an obtainment request for vaccination information corresponding to the vaccine recipient of the request source is transmitted to the vaccination information management server ASV under the control of the vaccination certification issuance processing unit 12B at step S41.

The control unit 1A of the vaccination information management server ASV determines, at step S36, whether or not the entry process of the post-vaccination information after the second vaccine is completed, and if yes, the control unit 1A monitors the reception of a vaccination information obtainment request at step S37. If a vaccination information obtainment request is received from the vaccination certification management server BSV, the vaccination completion information and the post-vaccination information of the target vaccine recipient are read out from the vaccination information storage unit 32A under the control of the vaccination information transfer processing unit 14A, and the read-out vaccination completion information and post-vaccination information are transferred to the vaccination certification management server BSV of the request source at step S38. At this time, the basic information of the vaccine recipient may also be transferred along with the vaccination completion information and the post-vaccination information.

When the vaccination completion information and the post-vaccination information are obtained under the control of the vaccination certification issuance processing unit 12B at step S41, the control unit 1B of the vaccination certification management server BSV first determines, based on the consent form information managed by the authentication server NSV, whether or not the vaccine recipient has previously consented to the issuance of vaccination certification data and the use of this data by a third party, at step S42. If the consent of the vaccine recipient is confirmed, the vaccination certification data is generated on the basis of the obtained vaccination completion information and post-vaccination information at step S43.

Here, the vaccination certification data includes, for example, the vaccination completion information of each of the first and second vaccinations, such as a vaccination date and time, vaccination site, the name of the vaccine recipient, a vaccine type, and a vaccine lot number for each vaccination. The vaccination certification data also includes information indicating the adverse reaction status and the like, which can be estimated by analyzing the post-vaccination information after the first and second vaccinations, the seriousness of the symptoms, the date and time of the onset, and the like. The vaccination certification data may also include a certification serial number, an issue date, start and end dates of the valid term, and electronic signature data of the vaccination certification management server BSV.

The vaccination certification issuance processing unit 12B stores the generated vaccination certification data in the vaccination certification storage unit 31B in association with the unique identification information of the vaccine recipient, and thereafter transmits the vaccination certification data from the communication I/F 4B to the vaccine recipient terminal UT of the request source. On the other hand, the control unit 1D of the vaccine recipient terminal UT receives the vaccination certification data under the control of the vaccination certification obtainment processing unit 13D at step S18 and stores the received vaccination certification data in the vaccination certification storage unit 33D.

Upon issuance of the vaccination certification data, the control unit 1B of the vaccination certification management server BSV instructs the trail management system BC to generate a token having management information of the vaccination certification data as an attribute value, under the control of the token registration processing unit 13B at step S44. Upon receipt of the generation instruction, the trail management system BC generates a token having an attribute value that corresponds to a parameter described in the management information by the contract, and stores the generated token as a transaction in association with the address of the vaccine recipient.

Examples for an attribute value of the token may include a token ID represented by a serial number or the like of the vaccination certification data, a vaccine recipient address and a vaccination certification issuer address on the trail management system BC, a storage destination URL of the vaccination certification data, a hash value of the vaccination certification data file, an expiration date of the vaccination certification data, and a token status (valid/invalid (e.g., expired)).

The vaccine recipient address represents an address value on the trail management system BS, which is paired with a secret key necessary for use of the trail management system BC by the vaccine recipient. The vaccination certification issuer address represents an address value on the trail management system BS, which is paired with a secret key necessary for use of the trail management system BC by the vaccination certification management server BSV that serves as an issuer of the vaccination certification data. The storage destination URL of the vaccination certification data represents the URL of the vaccination certification management server BSV for accessing the vaccination certification data. The hash value of the vaccination certification data file represents a hash function value for securing the authenticity of the vaccination certification data.

### (8) Submission and confirmation of vaccination certification data

In the first embodiment, an example will be described in which the vaccine recipient himself/herself submits vaccination certification data to a submission target organization (e.g., employer) in a face-to-face situation. The submission target organization may be various facilities such as a health department, a school, a library, a museum, a gymnasium, a meeting hall, a worship facility, an accommodation facility, a medical institution, a station, and an airport. Alternatively, it may be event sites such as a movie theater, a playhouse, and a stadium, or commercial facilities such as a travel agency, a shop, and a fitness center.

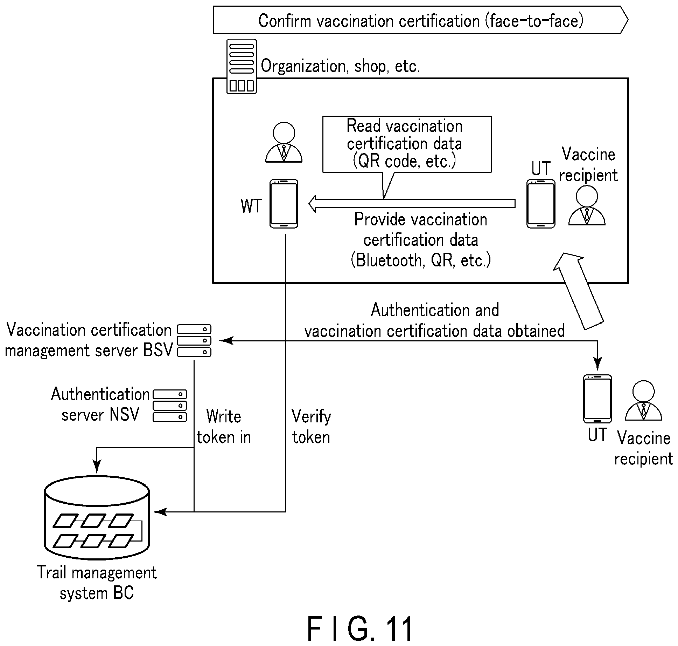

FIG. 11 is a sequence diagram showing a flow of processing from submission to confirmation of vaccination certification data according to the first embodiment.

After obtaining vaccination certification data from the vaccination certification management server BSV, the vaccine recipient visits the organization and submits this vaccination certification data to the organization using his/her own vaccine recipient terminal UT in a face-to-face situation. Specifically, the vaccination certification data is displayed on the vaccine recipient terminal UT so that the displayed vaccination certification data can be read by the organization terminal WT, as a result of which the vaccination certification data will be submitted to the organization.

Upon detection of an operation for presenting the vaccination certification data at step S19, the control unit 1D of the vaccine recipient terminal UT reads, under the control of the vaccination certification submission processing unit 14D, the vaccination certification data from the vaccination certification storage unit 33D at step S20, and displays the read-out vaccination certification data on the display unit 61D via the input/output I/F 5D. Here, the vaccination certification data may be represented as code data such as a bar code or a QR code. A hash value necessary for verification of the vaccination certification data is attached to this vaccination certification data.

On the organization side, the code data of the vaccination certification data such as a QR code displayed on the vaccine recipient terminal UT is read with an optical character reader (OCR) and imported into the organization terminal WT. If the organization terminal WT is a smartphone or any other terminal provided with a QR code reading function, an OCR reader is not required.

Upon obtainment of the vaccination certification data, the organization sends a request for verification of the obtained vaccination certification data from the organization terminal WT to the trail management system BC. For example, an address corresponding to the vaccine recipient in the trail management system BC is designated on the organization terminal WT by using a secret key obtained in advance from the vaccine recipient terminal UT, and a token verification request is transmitted. Upon receiving the verification request, the trail management system BC checks, for example, the hash value of the obtained vaccination certification data against the hash value of the token. Then, the information indicating the checking result is returned to the organization terminal WT of the request source as information indicating the verification result. The organization thereby confirms the authenticity of the vaccination certification data submitted by the vaccine recipient, i.e., whether or not tampering or the like has been performed.

### (Functions and Effects)

As described above, according to the first embodiment, the following functions and effects can be achieved.
(1) In the platform PF, the vaccination completion information transmitted from the medical service terminal MT in accordance with the administered vaccination is obtained and stored, and the post-vaccination information transmitted from the vaccine recipient terminal UT at a predetermined timing after the vaccination is obtained and stored. Upon transmission of a vaccination certification obtainment request from the vaccine recipient terminal UT, the platform PF creates vaccination certification data in which both the vaccination completion information and the post-vaccination information are reflected, and transmits the vaccination certification data to the vaccine recipient terminal UT of the request source.
   Therefore, vaccination certification data can be provided in which the post-vaccination information indicating any change in the conditions after the vaccination as well as the vaccination completion information indicating the fact of the vaccination having been administered is reflected. The vaccination certification data therefore allows the vaccine recipient to correctly ascertain his/her vaccination history.
(2) A token that indicates the history of changes in the created vaccination certification data from its issuance to use, changes of attribute values, and the like, is managed in the trail management system BC using a blockchain platform. If vaccination certification data is submitted from a vaccine recipient terminal UT to an organization terminal WT, upon which a verification request for the submitted vaccination certification data is sent from the organization terminal WT, verification of the vaccination certification data is conducted based on the token in the trail management system BC, and the verification result is reported to the organization terminal WT of the request source.
   Therefore, even if an unauthorized operation such as tampering is performed on the vaccination certification data, such an unauthorized operation can be reliably detected, and high reliability of the vaccination certification data can be maintained.
(3) At the time of the registration of the vaccination completion information, a consent form of the vaccine recipient regarding the use of the vaccination certification data by a third party is registered as consent confirmation information in the authentication server NSV. If permission or prohibition of the use of the vaccination certification data by the third party is confirmed based on the consent confirmation information, the issuance and transmission processes of the vaccination certification data are allowed.

This can obviate the risk that the vaccination certification data will be used by a third party without the consent of the vaccine recipient.

### <Second Embodiment>

In the first embodiment, after a vaccine recipient obtains the vaccination certification data, the obtained vaccination certification data is transferred in a face-to-face manner from the vaccine recipient terminal UT to the organization terminal WT of a submission target organization, and verification of the vaccination certification data is requested from the organization terminal WT to the trail management system BC to confirm whether or not the vaccination certification data is authentic.

In contrast, according to the second embodiment of the present invention, the vaccine recipient transmits a vaccination certification data submission request from the vaccine recipient terminal UT to the vaccination certification management server BSV for online submission of the vaccination certification data to the organization. In accordance with this request, the organization terminal WT accesses the vaccination certification management server BSV on behalf of the vaccine recipient to obtain the vaccination certification data of the vaccine recipient, and sends a request for the verification of the obtained vaccination certification data to the trail management system BC in order to confirm the authenticity of the vaccination certification data.

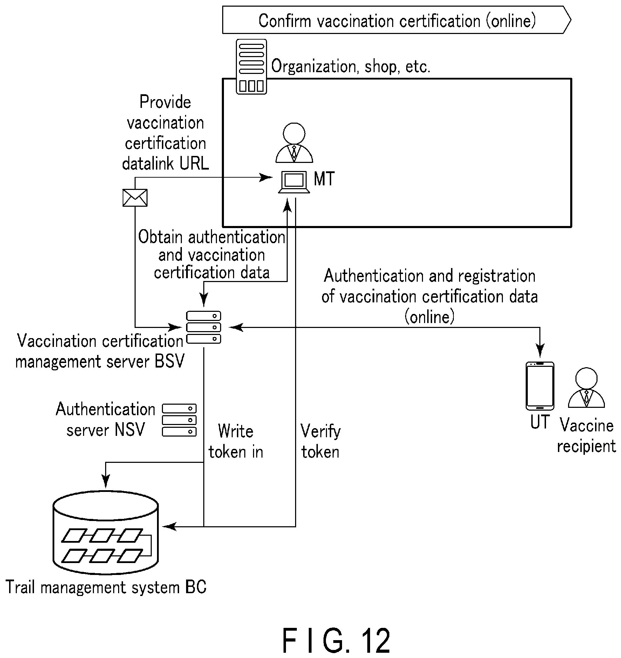

FIG. 12 is a sequence diagram showing a flow of the vaccination certification confirmation process according to the second embodiment of the present invention.

The functions of the management servers ASV and BSV of the platform PF and the trail management system BC are basically the same as those of the first embodiment, and therefore the description is omitted here.

In FIG. 12, first, the vaccine recipient transmits a vaccination certification data submission request from his/her own vaccine recipient terminal UT to the vaccination certification management server BSV of the platform PF via the network NW. This submission request includes the unique identification information and authentication information of the vaccine recipient who is the submitter of the vaccination certification data, and the address information of the organization terminal WT to which the vaccination certification data will be submitted. The vaccination certification management server BSV executes an authentication process upon the vaccine recipient based on the authentication information of the vaccine recipient managed by the authentication server NSV, and then transmits a vaccination certification obtainment request including a URL indicating the storage location of the vaccination certification data of the vaccine recipient to the organization terminal WT, for example, by electronic mail. For this obtainment request, communication means other than electronic mail such as SNS and SMS may be used instead.

If the organization terminal WT accesses the URL of the vaccination certification management server BSV in response to the vaccination certification obtainment request, the vaccination certification management server BSV first obtains the consent form information of the vaccine recipient, who is the transmission source of the submission request, from the authentication server NSV, and obtains the vaccination completion information and the post-vaccination information of this vaccine recipient from the vaccination information management server ASV. Then, whether or not the vaccine recipient consents to the third party's use of the vaccination certification data is determined based on the consent form information, and if the consent is confirmed, the vaccination certification data of the vaccine recipient is created from the vaccination completion information and the post-vaccination information. The created vaccination certification data is transmitted to the organization terminal WT.

Further, the vaccination certification management server BSV instructs the trail management system BC to generate a token of the created vaccination certification data. Upon receipt of this instruction, the trail management system BC generates a token of the vaccination certification data by contract, and stores the generated token as a transaction at an address corresponding to the vaccine recipient.

Upon receipt of the vaccination certification data from the vaccination certification management server BSV, the organization terminal WT transmits a token verification request for the received vaccination certification data to the trail management system BC. In response to this token verification request, the trail management system BC verifies the authenticity of the vaccination certification data based on the hash value included in the corresponding token stored in the transaction, and returns the verification result to the organization terminal WT of the request source.

According to the second embodiment of the present invention, when the vaccine recipient submits his/her own vaccination certification data to an organization, the vaccine recipient terminal UT transmits a vaccination certification data submission request that designates the submission target organization terminal WT, to the vaccination certification management server BSV via the network NW. Then, the organization terminal WT obtains the vaccination certification data of the vaccine recipient from the vaccination certification management server BSV on behalf of the vaccine recipient, and the trail management system BC verifies the obtained vaccination certification data.

As a result, even when the submission target organization for the vaccination certification data is located at a remote place or when it is difficult for the vaccine recipient to submit his/her own vaccination certification data to the organization in a face-to-face manner, the vaccine recipient can send to the vaccination certification management server BSV a request for submission of the vaccination certification data to the organization so that the vaccination certification data of the vaccine recipient can be submitted from the vaccination certification management server BSV to the submission target organization, in place of the vaccine recipient.

In this case also, a token of the vaccination certification data generated in the vaccination certification management server BSV is generated by the contract of the trail management system BC and stored as a transaction. In this manner, the organization can verify the obtained vaccination certification data with the trail management system BC.

### <Other Embodiments>

(1) In the first embodiment, the vaccination completion information, the information on the inquiry sheet, and the information on the consent form regarding a vaccine recipient at the time of vaccination are transmitted from the medical service terminal MT to the vaccination information management server ASV or the authentication server NSV of the platform PF and registered therein. The present invention is not limited thereto, however. For instance, the vaccination completion information, the inquiry sheet information, and the consent form information may be input or created on the vaccine recipient terminal UT, and transmitted from the vaccine recipient terminal UT to the vaccination information management server ASV or the authentication server NSV so as to be registered.
   If this is the case, for a vaccine recipient who does not own a vaccine recipient terminal UT, a local government, a medical institution, or a pharmaceutical manufacturer may prepare a tablet terminal or the like to share at a vaccination site. The vaccine recipient may use this shared terminal as a vaccine recipient terminal to input the vaccination completion information, the medical inquiry information, and the consent form information, and transmit them to the vaccination information management server ASV or the authentication server NSV.
(2) If the vaccine recipient owns a personal folder that can be used for arbitrary purposes on a website run by a communication carrier or a service carrier, a storage area referred to as a vaccination record or the like may be created in this personal folder. The vaccination completion information, the medical inquiry information, and the consent form information of the vaccine recipient may be registered in this vaccination record for each time of vaccination, and may be transferred from the vaccination record to the vaccination information management server ASV or the authentication server NSV of the platform PF.
(3) In the first embodiment, the transfer process of the vaccination completion information from the vaccine recipient terminal UT to the organization terminal WT is performed by optically reading the code data of the vaccination certification data, such as a bar code or a QR code. The present invention is not limited thereto, however, and for example, the vaccination certification data constituted by text data or binary data and stored in the vaccine recipient terminal UT may be transferred from the vaccine recipient terminal UT to the organization terminal WT using a wireless interface adopting a low-power wireless data transmission standard such as Bluetooth.
(4) In the first embodiment, the case where vaccination has been performed two times has been described as an example. The present invention, however, is applicable to the case where vaccination has been performed one time or three or more times. In addition, in the above-described embodiments, the case of vaccination has been described as an example. The present invention, however, is not limited thereto and is applicable to the case of clinical testing performed by a pharmaceutical company in the process of developing new medicines or the like other than vaccines. That is, the type of medicine is not limited to a vaccine, and may be any other pharmaceutical substances.
(5) In addition, various modifications can be made to the configuration of the platform, the functional configuration, processing procedure, and processing details of each of the servers included in the platform, the functional configuration of the vaccine recipient terminal, the processing procedure and processing details for obtaining the vaccination completion information, post-vaccination information, and vaccination certification information, the use of the vaccination certification information, and the like without departing from the scope of the present invention.

The programs according to the present embodiments may be transferred in a state of being stored in an electronic device or in a state of not being stored in an electronic device. In the latter case, the program may be transferred via a network or may be transferred in a state of being stored in a storage medium. The storage medium is a nontransitory tangible medium, and is a computer-readable medium. The storage medium may be any medium that can store a program and can be read by a computer, such as a CD-ROM or a memory card, and may be in any form.

The embodiments of the present invention have been described in detail above. The foregoing description is merely examples of the present invention in all respects. Various improvements and modifications can be added without departing from the scope of the invention. In other words, a specific configuration according to the embodiment may be adopted as appropriate in implementation of the present invention.

The present invention is not limited to the above-described embodiments as is, and can be embodied by modifying the structural components within a range that does not depart from the gist of the present invention at the implementation stage. In addition, various inventions may be constituted by appropriately combining multiple components disclosed in the embodiments. For example, some of the components may be deleted from the entirety of the components shown in each embodiment. Furthermore, the components of different embodiments may be suitably combined.

### REFERENCE SIGNS LIST

PF Medication information management apparatus (platform)
ASV Vaccination information management server
BSV Vaccination certification management server
NSV Authentication server
BC Trail management system
UT1 to UTn Vaccine recipient terminal
MT Medical service terminal
WT Organization terminal
NW Network
1A, 1B, 1D Control unit
2A, 2B, 2D Program storage unit
3A, 3B, 3D Data storage unit
4A, 4B, 4D Communication I/F
5D Input/output I/F
6D Input/output device
61D Display unit
62D Input unit
11A Vaccine recipient management processing unit
12A Vaccination completion information obtainment processing unit
13A Post-vaccination information obtainment processing unit
14A Vaccination information transfer processing unit
11B Vaccination information obtainment processing unit
12B Vaccination certification issuance processing unit
13B Token registration processing unit
11D Authentication/consent processing unit
12D Post-vaccination information entry processing unit
13D Vaccination certification obtainment processing unit
14D Vaccination certification submission processing unit
31A Vaccine recipient management information storage unit
32A Vaccination information storage unit
31B Vaccination certification storage unit
31D Authentication/consent confirmation information storage unit
32D Post-vaccination information storage unit
33D Vaccination certification storage unit

## Claims

1. A medication information management apparatus for allowing for information data transmission via a network between a user terminal used by a target recipient of medication and an organization terminal used by an organization to which medication certification information relating to the medication is to be submitted, the medication information management apparatus comprising:
a medication completion information obtainment processing unit configured to obtain medication completion information that indicates a fact of the medication having been administered to the target recipient and store the obtained medication completion information in association with unique identification information of the target recipient;
a post-medication information obtainment processing unit configured to obtain post-medication information that indicates a condition of the target recipient after the medication from the user terminal and store the obtained post-medication information in association with the medication completion information;
a medication certification information creation processing unit configured to create the medication certification information based on the medication completion information and the post-medication information; and
a submission control processing unit configured to control a process of submitting the medication certification information from the target recipient to the organization.

2. The medication information management apparatus according to claim 1, further comprising:
a processing unit configured to conduct an authentication process upon the target recipient and a consent process for confirming consent of the target recipient to use of the medication certification information by the organization.

3. The medication information management apparatus according to claim 1, wherein
the submission control processing unit further includes a verification processing unit configured to implement a verification procedure on the medication certification information submitted to the organization.

4. The medication information management apparatus according to claim 3, further comprising:
a trail information management processing unit configured to generate and store trail information in which a history of changes to the medication completion information and the post-medication information is recorded,
wherein the submission control processing unit comprises:
a processing unit for transmitting, in response to a request for obtaining the medication certification information that has been sent from the user terminal, the medication certification information to the user terminal of a request source;
a processing unit for receiving a verification request of the medication certification information transmitted from the organization terminal after a process of transferring the medication certification information from the user terminal to the organization terminal; and
a processing unit for conducting a verification upon the medication certification information by collating the verification request with the trail information and returning information indicating a result of the verification to the organization terminal.

5. The medication information management apparatus according to claim 3, further comprising:
a trail information management processing unit configured to generate and store trail information in which a history of changes to the medication completion information and the post-medication information is recorded,
wherein the submission control processing unit comprises:
a processing unit for receiving an obtainment request of the medication certification information transmitted from the organization terminal after a confirmation request of the medication certification information transmitted from the user terminal to the organization terminal;
a processing unit for transmitting, in response to the obtainment request, the medication certification information to the organization terminal of a request source;
a processing unit for receiving a verification request of the medication certification information transmitted from the organization terminal; and
a processing unit for conducting a verification upon the medication certification information by collating the verification request with the trail information, and transmitting information indicating a result of the verification to the organization terminal.

6. The medication information management apparatus according to claim 4, wherein
the trail information management processing unit manages the trail information using a blockchain platform in which a plurality of distributed ledgers are connected via a peer-to-peer (P2P) network.

7. A medication information management method executed by an information management apparatus that allows for information data transmission via a network between a user terminal used by a target recipient of medication and an organization terminal used by an organization to which medication certification information relating to the medication is to be submitted, the medication information management method comprising:
obtaining medication completion information that indicates a fact of the medication having been administered to the target recipient and storing the obtained medication completion information in association with unique identification information of the target recipient;
obtaining post-medication information that indicates a condition of the target recipient after the medication from the user terminal and storing the obtained post-medication information in association with the medication completion information;
creating the medication certification information based on the medication completion information and the post-medication information; and
controlling a process of submitting the medication certification information from the target recipient to the organization.

8. A program storage medium for storing a program that causes a processor of the medication information management apparatus according to any one of claims 1 to 6 to execute processes that are performed by the processing units of the medication information management apparatus.
